# EUROPÄISCHE PATENTSCHRIFT

(11) **EP 1 252 864 B1**
(45) Veröffentlichungstag und Bekanntmachung des Hinweises auf die Patenterteilung: **10.01.2007**
(21) Anmeldenummer: 02004363.4
(22) Anmeldetag: 04.03.2002
(51) Int. Cl.: A61B 17/68, A61B 17/92

(54) **Selbsthaltendes Implantat und Vorrichtung zum Befestigen des Implantats**
Self-retaining implant and device for attaching it
Implant auto-rétentif et dispositif pour son montage

(30) Priorität: 24.04.2001 DE 20107039 U; 01.08.2001 US 921233
(43) Veröffentlichungstag der Anmeldung: 30.10.2002
(60) Teilanmeldung: 06019502.1
(73) Patentinhaber: Stryker Leibinger GmbH & Co. KG, 79111 Freiburg (DE)
(72) Erfinder: Knöpfle, Christian, 78166 Donaueschingen (DE); Frank, Thorsten, 78532 Tuttlingen (DE); Greiner, Karl, 78570 Mühlheim (DE); Eckerle, Hans-Urs, 79117 Freiburg (DE)
(74) Vertreter: Röthinger, Rainer

(56) Entgegenhaltungen:
- US-A- 5 741 268
- US-A- 5 964 762
- US-A- 6 010 513
- US-B1- 6 168 596
- US-B1- 6 190 389

## Beschreibung

Die Erfindung betrifft ein Implantat zur Festlegung eines Knochendeckels oder Knochenfragments, insbesondere zur Festlegung eines craniotomierten Knochendeckels oder eines Schädelknochenfragments an der Schädelkalotte.

Im Rahmen von Schädeloperationen werden häufig Knochendeckel, d.h. plattenförmige Knochenteile, aus dem Schädelknochen herausgesägt. Diese Knochendeckel müssen nach Abschluß der Operation wieder in der entstandenen Öffnung der Schädeldecke fixiert werden, damit die Knochendeckel in ihrer alten Position wieder einheilen können. Eine vergleichbare Problemstellung tritt auf, wenn Schädelknochenfragmente im Bereich der Schädeldecke fixiert werden müssen.

Zur Festlegung von Knochendeckeln oder Schädelknochenfragmenten wurden unterschiedliche Implantate vorgeschlagen. So sind Implantate bekannt, welche zwei Schraubenlöcher aufweisen. In einem ersten Schritt werden derartige Implantate mittels Schrauben z.B. an dem festzulegenden Knochendeckel befestigt. Anschließend werden die mit dem Knochendeckel verschraubten Implantate zusätzlich am Schädelknochen festgeschraubt. Aufgrund der Tatsache, daß jedes einzelne Implantat mittels je einer Schraube zunächst am Knochendeckel und anschließend am Schädelknochen befestigt werden muß, ist das Festlegen des Knochendeckels sehr zeitaufwendig. Um den mit der Festlegung eines Knochendeckels verbundenen Zeitaufwand zu reduzieren, wurden selbsthaltende Implantate vorgeschlagen.

Ein derartiges, selbsthaltendes Implantat ist beispielsweise aus der DE 199 07 354 A1 bekannt. Bei diesem Implantat handelt es sich um ein klammerartiges Element, welches auf den festzulegenden Knochendeckel aufgesteckt wird. Das Implantat umfaßt hierzu zwei beabstandet voneinander angeordnete Anlegearme, welche im aufgesteckten Zustand des Implantats den Knochendeckel umgreifen. Ein erster der beiden Anlegearme kontaktiert dabei die Oberseite des Knochendeckels und ein zweiter der beiden Anlegearme kontaktiert die Unterseite.

Nachteilig bei einem derartigen, klammerartigen Implantat ist dessen umständliche Handhabung. Die umständliche Handhabung ist in erster Linie darauf zurückzuführen, daß unterschiedliche Knochendeckel nicht immer die selbe Stärke aufweisen und auch die Stärke ein und desselben Knochendeckels variieren kann. Nachteilig ist weiterhin, daß der auf der Unterseite des Knochendeckels anliegende Anlegearm bei festgelegtem Knochendeckel auf den Hirnhautlappen aufliegt und diese unter Umständen verletzt.

Aus der US 6,190,389 B1 ist ein selbsthaltendes Implantat bekannt, das mittels einer lateral in den Knochendeckel einzutreibenden Spitze am Knochendeckel befestigt wird. Das Implantat besitzt einen mit einem Schraubenloch versehenen Auflagearm, um mittels Schrauben am Schädelknochen befestigt zu werden.

Die US 6,168,596 B1 beschreibt ein weiteres Implantat mit einem sich über den Spalt zwischen Knochendeckel und Schädelknochen erstreckenden Auflagearm. Das Implantat wird mittels Knochenschrauben am Knochendeckel befestigt und verhakt sich mittels eines Widerhakens lateral am Schädelknochen.

Der Erfindung liegt die Aufgabe zugrunde, ein Implantatsystem anzugeben, welches ein selbsthaltendes Implantat mit einer vereinfachten Handhabbarkeit umfaßt.

Diese Aufgabe wird erfindungsgemäß gelöst durch ein Implantat gemäß Anspruch 1.

Das selbsthaltende Implantat besitzt ein Auflageelement mit einer Oberseite und einer dem Knochendeckel oder dem Knochenfragment zugewandten Unterseite. Auf der Unterseite des Auflageelements ist ein Fortsatz angeordnet, der mindestens eine, sich in Richtung auf den Knochendeckel erstreckende Spitze trägt. Die Spitze wird zur selbsthaltenden Verankerung des Implantats lateral in den Knochendeckel oder das Knochenfragment eingetrieben.

Eine Eintreibvorrichtung für das Implantat umfaßt gemäß einer ersten Variante eine Aufnahme für das selbsthaltende Implantat sowie einen an die Aufnahme koppelbaren Eintreibmechanismus zum Eintreiben des Implantats, vorzugsweise der mindestens einen Spitze des Implantats, lateral in den Knochendeckel oder das Knochenfragment. Der Eintreibmechanismus kann hierbei mit einer mit der Aufnahme kraftübertragend gekoppelten Krafteinleitungsfläche derart gekoppelt sein, daß die Eintreibkraft vom Eintreibmechanismus in die Krafteinleitungsfläche, von dieser in die Aufnahme und von der Aufnahme in das Implantat übertragen wird. Gemäß einer zweiten Variante der Eintreibvorrichtung ist kein Eintreibmechanismus vorhanden. In diesem Fall wird beispielsweise unmittelbar mittels eines hammerartigen Werkzeugs die Eintreibkraft in die mit der Aufnahme kraftübertragend gekoppelte Krafteinleitungsfläche eingeleitet.

Das erfindungsgemäße, selbsthaltende Implantat wird nicht mittels Aufschiebens am Knochendeckel befestigt, sondern mittels einer oder mehrerer in den Knochendeckel oder das Knochenfragment eingetriebener Spitzen. Das Eintreiben der mindestens einen Spitze des Implantats erfolgt vorzugsweise unter Verwendung einer Eintreibvorrichtung, welche zu diesem Zweck einen geeigneten Eintreibmechanismus umfassen kann. In der Praxis hat sich herausgestellt, daß ein Eintreiben des selbsthaltenden Implantats, insbesondere bei Verwendung einer Eintreibvorrichtung, aus Gesichtspunkten der Handhabung besonders vorteilhaft ist. Die aus dem Eintreiben der Spitze des Implantats in z.B. den spongiösen oder kortikalen Bereich des Knochendeckels oder des Knochenfragments resultierende Verbindung zwischen dem Implantat und dem Knochendeckel oder dem Knochenfragment ist äußerst stabil und zuverlässig.

Die mindestens eine Spitze des selbsthaltenden Implantats ist bezüglich des Auflageelements derart angeordnet, daß sie zuverlässig in den Knochendeckel oder das Knochenfragment eingetrieben werden kann und eine gute Verankerung des Implantats gewährleistet. Zu diesem Zweck erstreckt sich die Spitze vorzugsweise im wesentlichen parallel zum Auflageelement. Die Spitze kann jedoch bezüglich des Auflageelements auch geneigt sein, sofern eine ausreichende Eintreibbarkeit gewährleistet ist. Der Übergang zwischen dem Fortsatz und der am Fortsatz angeordneten Spitze kann kontinuierlich sein, so daß keine definierte Grenze zwischen Fortsatz und Spitze erkennbar ist. Gemäß einer bevorzugten Ausführungsform steht die Spitze jedoch unter einem Winkel von beispielsweise 90° vom Fortsatz ab.

Der Fortsatz schließt mit dem Auflageelement zweckmäßigerweise einen Winkel zwischen 45° und 135° ein. Bevorzugt ist ein Fortsatz, welcher sich im wesentlichen rechtwinkelig zum Auflageelement erstreckt. In diesem Fall kann der Fortsatz als Anschlag fungieren, welcher ein definiertes Eintreiben der Spitze ermöglicht. Mit anderen Worten: sobald der Fortsatz in Anlage an laterale Bereiche des Knochendeckels oder des Knochenfragments gelangt, verhindert er ein weiteres Eintreiben der Spitze. Außerdem gestattet eine Analyse der Lage des Fortsatzes relativ zu einem lateralen Bereich des Knochendeckels oder des Knochenfragments eine visuelle Beurteilung, ob die Spitze des Implantats ausreichend tief eingetrieben wurde.

Gemäß einer bevorzugten Ausführungsform ist der Fortsatz derart relativ zum Auflageelement angeordnet, daß das Auflageelement und der Fortsatz eine im Querschnitt im wesentlichen T-förmige Struktur bilden. Das Auflageelement entspricht dabei dem Querbalken des T und der Fortsatz dessen Längsbalken. Der Fortsatz unterteilt das Auflageelement somit in zwei sich in entgegengesetzte Richtungen erstreckende Auflagearme. Ein erster der beiden Auflagearme verläuft in Richtung auf den Knochendeckel und ein zweiter der beiden Auflagearme in Richtung auf den Schädelknochen. Eine derartige Ausgestaltung des Implantats ist vorteilhaft, da der sich in Richtung auf den Knochendeckel erstreckende und auf der Knochendeckeloberseite aufliegende Auflagearm zusammen mit der in den spongiösen Bereich des Knochendeckels eingetriebenen Spitze eine besonders zuverlässige Befestigung des Implantats am Knochendeckel ermöglicht. Außerdem kann der mit dem Knochendeckel zusammenwirkende Auflagearm als Führung während des Positionierens sowie des Eintreibens des Implantats verwendet werden.

Das Auflageelement besitzt vorzugsweise eine flächige, z.B. eine streifenförmige Gestalt. Die Unterseite des Auflageelements kann zumindest in Bereichen konkav oder sphärisch gekrümmt sein, um eine gute Anpassung des Implantats an die Schädelrundung zu gewährleisten. Zweckmäßigerweise ist das Auflageelement des Implantats zumindest bereichsweise biegsam ausgestaltet, um eine individuelle Anpassung des Implantats an die Schädelrundung zu ermöglichen.

Die einzutreibende Spitze des Implantats ist z.B. an einem dem Auflageelement abgewandten Ende des Fortsatzes angeordnet. Die Spitze kann unterschiedlich ausgestaltet sein. So ist es beispielsweise möglich, eine Spitze in Gestalt eines oder mehrerer Dorne oder einer Sägezahnstruktur vorzusehen. Vorzugsweise besitzt die Spitze jedoch eine flächige, annähernd dreieckige Gestalt. Um das Eintreiben der Spitze zu erleichtern, kann die Spitze mit geschärften Kanten versehen werden. Die Länge der Spitze ist in weiten Bereichen frei wählbar, sofern eine ausreichend zuverlässige Verankerung des Implantats gewährleistet ist.

An seinem der Spitze entgegengesetzten Ende kann das Auflageelement ein Schraubenloch besitzen, um ein Festlegen des mit einem oder mehreren Implantaten versehenen Knochendeckels oder Knochenfragments mittels Schrauben am Schädelknochen zu ermöglichen. Bei einem Auflageelement mit zwei oder mehr Auflagearmen ist das Schraubenloch folglich in demjenigen Auflagearm ausgebildet, welcher mit dem Schädelknochen zusammenwirkt. Vorzugsweise besitzt das Auflageelement im Bereichs des Schraubenlochs eine lokal höhere Stärke. Dabei kann die Stärke des Auflageelements im Bereich des Schraubenlochs derart gewählt sein, daß der Schraubenkopf bereichsweise oder vollständig im Schraubenloch versenkbar ist. Dies ist aus kosmetischen Gründen vorteilhaft. In Bereichen des Auflageelements außerhalb des Schraubenlochs kann die Stärke des Auflageelements hingegen deutlich geringer gewählt werden. Das Innere des Schraubenlochs ist zweckmäßigerweise sphärisch gekrümmt, um das Versenken von Knochenschraubenköpfe mit einer korrespondierenden sphärischen Krümmung zu ermöglichen.

Das erfindungsgemäße Implantat kann auf unterschiedliche Weise in den Knochendeckel oder das Knochenfragment eingetrieben werden. Mittels der einen Eintreibmechanismus umfassenden Eintreibvorrichtung ist dies auf besonders einfache Weise möglich. Der Eintreibmechanismus kann derart ausgestaltet sein, daß er das Ausüben einer Schlagkraft auf die für das Implantat vorgesehene Aufnahme gestattet. Zu diesem Zweck kann der Eintreibmechanismus ein Schlagelement umfassen, welches gegen eine Federkraft verschiebbar ist. Die Federkraft ermöglicht es, das Schlagelement in Eintreibrichtung vorzuspannen, wobei die Schlagkraft durch einen plötzlichen Abbau der Vorspannung aktivierbar ist.

Vorzugsweise ist das Schlagelement von einem koaxial zur Aufnahme geführten, ersten Schlitten gebildet. Eine Führung für den ersten Schlitten kann beispielsweise von einem koaxial zur Aufnahme angeordneten Bolzen gebildet werden. Die Aufnahme kann derart mit dem Bolzen oder einer anderen ortsfesten Komponente der Eintreibvorrichtung verbunden sein, daß die Aufnahme in axialer Richtung begrenzt beweglich ist.

Die Eintreibvorrichtung umfaßt zweckmäßigerweise einen Betätigungsmechanismus für den Eintreibmechanismus. Im Fall eines Eintreibmechanismus, welcher ein gegen eine Federkraft verschiebbares Schlagelement besitzt, wird die zum Verschieben des Schlagelements benötigte Kraft vorzugsweise mittels des Betätigungsmechanismus aufgebracht. Der Betätigungsmechanismus kann zu diesem Zweck mit Hilfe eines Elektromotors oder eines oder mehrerer Finger betätigbar sein.

Zum Koppeln des Betätigungsmechanismus mit dem Schlagelement, d.h. z.B. zum Vorspannen des Schlagelements, kann eine Koppeleinrichtung vorgesehen werden. Die Koppeleinrichtung umfaßt beispielsweise einen Mitnehmer für das Schlagelement. Bei Vorhandensein einer Koppeleinrichtung ist zweckmäßigerweise eine korrespondierende Entkoppeleinrichtung vorhanden, welche zum Aktivieren der Schlagkraft ein Entkoppeln von Betätigungsmechanismus und Schlagelement gestattet.

Gemäß einer besonders bevorzugten Ausführungsform weist die Eintreibvorrichtung einen pistolenartigen Aufbau auf. Eine derartige Formgebung gestattet ein besonders ergonomisches und sicheres Eintreiben der Implantate. Andere Formgebungen der Eintreibvorrichtung sind jedoch ebenfalls denkbar. So kann die Eintreibvorrichtung beispielsweise eine im wesentlichen zylindrische Gestalt besitzen.

Eine Eintreibvorrichtung mit pistolenartigem Aufbau besitzt typischerweise einen Pistolenkörper und einen Pistolenlauf. Der Pistolenkörper kann einen Pistolenhandgriff sowie ein mit dem Handgriff verbundenes Gehäuse zur Aufnahme des Eintreibmechanismus besitzen. Der Betätigungsmechanismus für den Eintreibmechanismus kann als fingerbetätigbarer Pistolenabzug ausgebildet sein. Vorzugsweise ist der Pistolenabzug mit einem gegen eine Federkraft verschiebbaren, zweiten Schlitten gekoppelt. Die vorstehend beschriebene Koppeleinrichtung kann in diesem Fall funktionell zwischen dem ersten und dem zweiten Schlitten angeordnet sein.

Bei einer pistolenartigen Ausgestaltung der Eintreibvorrichtung ist es möglich, die Aufnahme für das Implantat nach Art eines Pistolenlaufs bezüglich des Pistolenkörpers anzuordnen. Eine derartige Anordnung der Aufnahme gestattet ein besonders einfaches Positionieren des einzutreibenden Implantats bezüglich des Knochendeckels oder des Knochenfragments.

Die Aufnahme kann mit einem Selbsthaltemechanismus für das Implantat versehen sein, so daß das Implantat unmittelbar aus einem hierfür vorgesehenen Magazin mittels der Eintreibvorrichtung entnommen werden kann. Es kann daher während des gesamten Befestigungsvorgangs des Implantats darauf verzichtet werden, das Implantat mit den Fingern zu berühren. Dies ist aus ergonomischen Gesichtspunkten wünschenswert.

Nachfolgend wird ein Ausführungsbeispiel des Implantats und ein Ausführungsbeispiel der Vorrichtung anhand mehrerer Figuren näher erläutert. Es zeigt:
- Fig. 1: eine Seitenansicht eines Ausführungsbeispiels des erfindungsgemäßen Implantats;
- Fig. 2: eine Aufsicht auf die der Knochenplatte zugewandten Unterseite des Implantats gemäß Fig. 1;
- Fig. 3: eine Schnittansicht durch das Auflageelement des Implantats gemäß den Fig. 1 und 2 im Bereich eines Schraubenlochs;
- Fig. 4: eine Seitenansicht eines ersten Ausführungsbeispiels einer Eintreibvorrichtung;
- Fig. 5: einen Längsschnitt durch die Eintreibvorrichtung gemäß Fig. 4;
- Fig. 6: einen Querschnitt durch die Eintreibvorrichtung gemäß Fig. 4 entlang der Linie VI-VI gemäß Fig. 5;
- Fig. 7a und 7b: die Aufnahme für das Implantat in einer Aufsicht und einer Schnittansicht;
- Fig. 8: ein zweites Ausführungsbeispiel einer Eintreibvorrichtung in einer Aufsicht; und
- Fig. 9: eine Schrägansicht des eingetriebenen Implantats gemäß Fig. 1 nach dessen Befestigung im Schädelknochen.

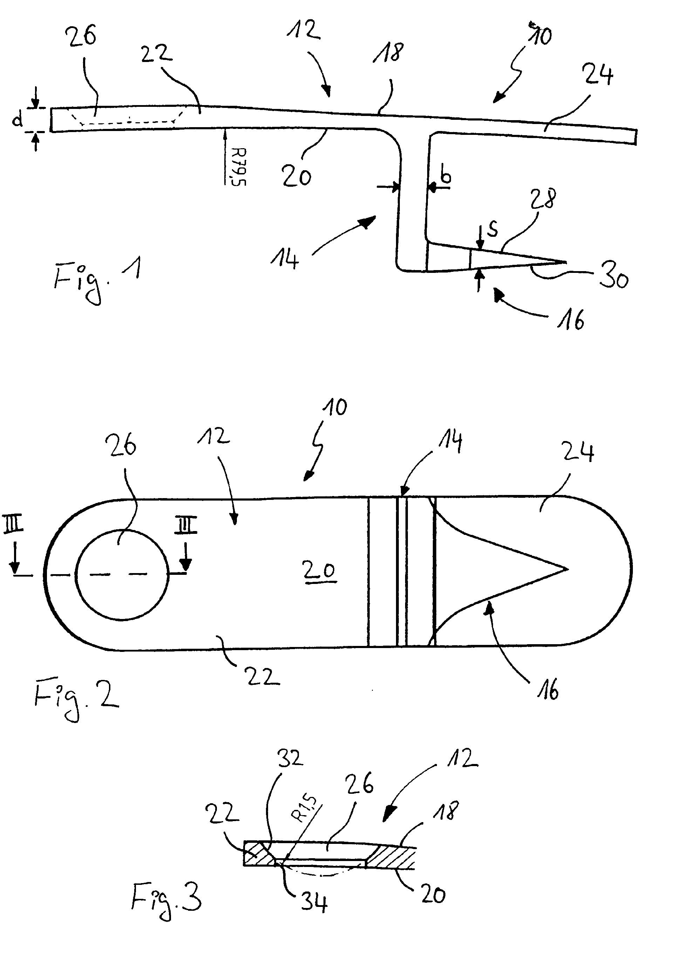

In Fig. 1 ist ein Ausführungsbeispiel eines selbsthaltenden Implantats 10 gemäß der Erfindung in einer Seitenansicht dargestellt. Das Implantat 10 umfaßt ein Auflageelement 12, einen mit dem Auflageelement 12 verbundenen Fortsatz 14 und eine mit dem Fortsatz 14 verbundene Spitze 16.

Das Auflageelement 12 besitzt eine Oberseite 18 und eine Unterseite 20. Die Unterseite 20 ist nach der Implantation einem in Fig. 1 nicht dargestellten Knochendeckel oder Knochenfragment sowie einem in Fig. 1 ebenfalls nicht dargestellten Schädelknochen zugewandt. Um eine möglichst spielfreie Auflage der Unterseite 20 zu gewährleisten, ist diese sphärisch gekrümmt. Der Krümmungsradius R beträgt 79,5 mm.

Das Auflageelement 12 setzt sich aus zwei Auflagearmen 22, 24 zusammen. Der in Fig. 1 linke Auflagearm 22 liegt im implantierten Zustand des Implantats 12 auf dem Schädelknochen auf und der in Fig. 1 rechte Auflagearm 24 auf z.B. dem Knochendekkel. Das Längenverhältnis zwischen dem linken Auflagearm 22 und dem rechten Auflagearm 24 läßt sich mittels der Position des Fortsatzes 14 relativ zu den beiden Auflagearmen 22, 24 variieren. In der Darstellung gemäß Fig. 1 besitzt der linke Auflagearm 22 eine etwas größere Länge als der rechte Auflagearm 24.

Der linke Arm 22 ist an seinem der Spitze 16 abgewandten Ende mit einem Schraubenloch 26 versehen. Im Bereich des Schraubenlochs 26 besitzt das Auflageelement 12 seine maximale Stärke d von ungefähr 0,5 mm. Diese Stärke wurde gewählt, um ein kosmetisch vorteilhaftes Versenken eines Knochenschraubenkopfes im Auflageelement 12 zu ermöglichen. In vom Schraubenloch 26 beabstandeten Bereichen besitzt das Auflageelement 12 eine geringere Stärke von typischerweise 0,3 mm. Diese geringere Stärke ist derart gewählt, daß das Auflageelement in Bereichen außerhalb des Schraubenlochs geringfügig gebogen werden kann. Mittels Biegens des Auflageelements 12 kann eine individuelle Anpassung der Unterseite 20 an die Krümmung des Knochendeckels oder Schädelknochens erfolgen.

Der Fortsatz 14 des Implantats 10 ist an der Unterseite 20 des Auflageelements 12 befestigt und erstreckt sich im wesentlichen rechtwinkelig zum Auflageelement 12. Das Auflageelement 12 und der Fortsatz 14 bilden zusammen eine T-förmige Struktur. Dabei entspricht das Auflageelement 12 dem Querbalken der T-förmigen Struktur und der Fortsatz 14 dem Längsbalken.

Die Breite b des Fortsatzes 14 sollte gering sein, damit der Knochenspalt zwischen dem Knochendeckel oder dem Knochenfragment und dem Schädelknochen klein gehalten werden kann. Zweckmäßig sind Breiten b von weniger als einem Millimeter, beispielsweise von 0,6 mm. Mittels des Implantats 10 kann der Knochendeckel oder das Knochenfragment derart im Schädelknochen positioniert werden, daß der Knochenspalt entlang der Kontur des Knochendeckels oder des Knochenfragments konstant ist. Um ein schnelleres Anwachsen des Knochendeckels oder des Knochenfragments sicherzustellen, kann der Knochendeckel oder das Knochenfragment jedoch auch mittels des Implantats 10 in bereichsweisem Kontakt mit dem Schädelknochen festgelegt werden.

Wenn das Auflageelement 12 oder der Fortsatz 14 mit der Eintreibkraft, welche in Fig. 1 nach rechts wirkt, beaufschlagt wird, wird die Eintreibkraft vom Fortsatz 16 auf die Spitze 16 zu übertragen. Die Spitze 16 ist an demjenigen Ende des Fortsatzes 14 ausgebildet, welches dem Auflageelement 12 gegenüberliegt. In Fig. 1 ist deutlich zu erkennen, daß sich die Spitze senkrecht zum Fortsatz 14 und im wesentlichen parallel zum Auflageelement 12 erstreckt. Die Stärke s der Spitze 16 nimmt mit zunehmendem Abstand vom Fortsatz 14 allmählich ab. Die Kanten 28, 30 der Spitze 16 sind geschärft, um das Eintreiben der Spitze in den Knochendeckel oder das Knochenfragment zu erleichtern.

In Fig. 2 ist das Implantat 10 gemäß Fig. 1 in einer Aufsicht auf die Unterseite 20 des Auflageelements 12 dargestellt. Wie Fig. 2 entnommen werden kann, besitzt das Auflageelement 12 eine im wesentlichen streifenförmige Gestalt mit abgerundeten Enden. Die Spitze 16 ist dreieckig ausgestaltet und kann daher mit vergleichsweise geringer Eintreibkraft eingebracht werden. Alternative Ausgestaltungen der Spitze 16 sind ebenfalls denkbar. So könnte die Spitze 16 beispielsweise zusätzlich mit widerhakenartigen Strukturen versehen sein, um die Verankerung der Spitze 16 im spongiösen Bereich zu verbessern.

Fig. 3 zeigt einen Schnitt durch das in Fig. 2 dargestellte Implantat im Bereich des Schraubenlochs 26. Das Innere des Schraubenlochs 26 besitzt eine sphärisch gekrümmten Bereich 32, welcher in Richtung auf die Unterseite 20 in einen kurzen, zylindrischen Bereich 34 mündet. Der sphärisch gekrümmte Bereich 32 ermöglicht das Versenken von korrespondierend geformten Knochenschraubenköpfen im Auflageelement 12.

Das Implantat 10 gemäß den Fig. 1 bis 3 ist selbsthaltend, da es ohne Befestigungselemente wie Schrauben, Nägel, usw. an z.B. einem Knochendeckel befestigbar ist. Im Gegensatz zu den klammerartigen Implantaten des Stands der Technik ist die Haltekraft des Implantats 10 im wesentlichen unabhängig von der Stärke des Knochendeckels. So kann auch bei geringen Knochendeckelstärken eine feste Verankerung des erfindungsgemäßen Implantats gewährleistet werden. Das Implantat 10 wird lateral in den Knochendeckel oder in das Knochenfragment eingetrieben. Die Verankerung des Implantats 10 erfordert daher keine auf der Unterseite des Knochendeckels oder des Knochenfragments aufliegende Elemente, so daß eine Verletzung der Hirnhautlappen durch das Implantat 10 ausgeschlossen ist.

Die Länge des Fortsatzes 14, d.h. der Abstand zwischen der Spitze 30 und dem Auflageelement 12, ist typischerweise derart gewählt, daß das Implantat 10 bei Knochendeckelstärken bis 3,5 mm sicher verankerbar ist. Durch Bereitstellen von Implantaten 10 mit kürzeren Fortsätzen 14 können auch Knochendeckel mit einer Stärke von weniger als 3,5 mm festgelegt werden. Das Implantat 10 ist aus biokompatiblem TiAl6V4 hergestellt. Anstatt aus einem Metall kann das Implantat 10 jedoch auch ganz oder bereichsweise aus einem resorbierbaren Material bestehen.

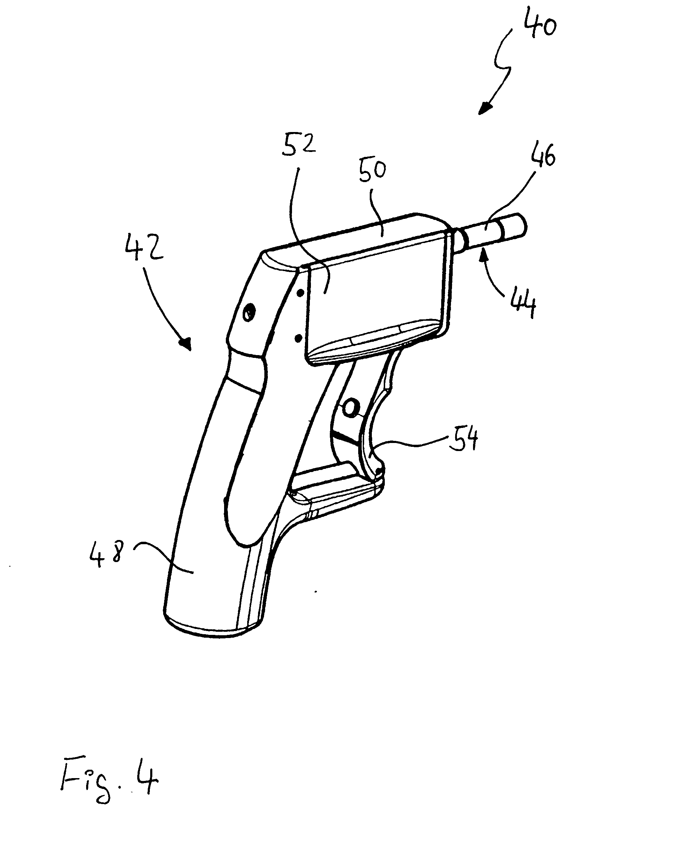

In Fig. 4 ist ein erstes Ausführungsbeispiel einer Eintreibvorrichtung 40 für das Implantat 10 gemäß den Fig. 1 bis 3 dargestellt. Die Eintreibvorrichtung 40 besitzt die Form einer Pistole mit einem Pistolenkörper 42 und einem Pistolenlauf 44. Der Pistolenlauf 44 wird von einer Aufnahme 46 für das einzutreibende Implantat gebildet. Der Pistolenkörper 42 setzt sich aus einem Handgriff 48 und einem Gehäuse 50 zusammen. Innerhalb des Gehäuses 50 ist der in Fig. 4 nicht dargestellte, mit der Aufnahme 46 zusammenwirkende Eintreibmechanismus angeordnet. Der Eintreibmechanismus gestattet das Einleiten einer Eintreibkraft in die Aufnahme 46.

Das Gehäuse 50 umfaßt zwei seitlich angeordnete und vollständig abnehmbare Gehäusedeckel. In der Ansicht gemäß Fig. 4 ist ein einziger Gehäusedeckel 52 zu erkennen. Die abnehmbaren Gehäusedeckel gestatten eine einfache Reinigung und Schmierung des Eintreibmechanismus im Anschluß an die Operation.

Für die Betätigung des Eintreibmechanismus ist ein Betätigungsmechanismus in Gestalt eines Pistolenabzugs 54 vorgesehen. Der Pistolenabzug 54 besitzt eine ergonomische Form, welche an eine Aktivierung mittels Zeigefinger und Mittelfinger oder mittels Mittelfinger und Ringfinger angepaßt ist.

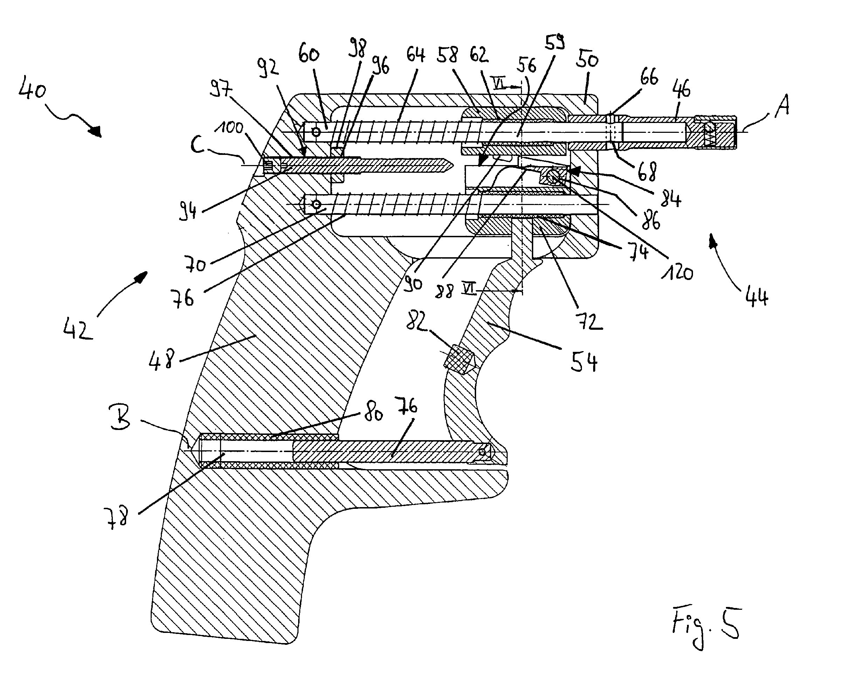

Fig. 5 zeigt einen Längsschnitt durch die Eintreibvorrichtung 40 gemäß Fig. 4 und insbesondere den Eintreibmechanismus 56 der Eintreibvorrichtung 40.

Der Eintreibmechanismus 56 ist als Schlagmechanismus ausgestaltet und umfaßt ein Schlagelement in Gestalt eines oberen Schlittens 58. Der obere Schlitten 58 besitzt eine zentrale Durchgangsöffnung 59, durch welche sich ein am Gehäuse 50 verankerter, ortsfester, oberer Führungsbolzen 60 erstreckt. Der obere Schlitten 58 ist mittels eines Kugellagers 62 entlang des oberen Führungsbolzens 60 verschiebbar geführt.

Der obere Führungsbolzen 60 ist radial außen von einer Schraubenfeder 64 umgeben, welche in der in Fig. 5 dargestellten Ausgangsstellung des Eintreibmechanismus 56 mit ihrem einen Ende am Gehäuse 50 und mit ihrem anderen Ende am oberen Schlitten 58 anliegt. Bei einer Bewegung des Schlittens entlang des oberen Führungsbolzens 60 in Fig. 5 nach links wird die Schraubenfeder 64 komprimiert. Gleichzeitig wird der obere Schlitten 58 in Fig. 5 nach rechts, d.h. in Eintreibrichtung, vorgespannt.

Der obere Führungsbolzen 60 ragt pistolenlaufartig teilweise aus dem Gehäuse 50 heraus. An seinem aus dem Gehäuse 50 herausragenden Ende ist der obere Führungsbolzen 60 mit der dieses Ende radial außen umgebenden Aufnahme 46 für das Implantat gekoppelt. Die Kopplung des oberen Führungsbolzens 60 mit der Aufnahme 46 geschieht mittels eines starr mit dem oberen Führungsbolzen 60 verbundenen Querbolzens 66. Der Querbolzen 66 ragt durch eine Öffnung 68 der Aufnahme 46. Die Öffnung 68 ist ein Längsloch mit einer ovalen Gestalt und ermöglicht daher eine begrenzte Bewegung der Aufnahme 46 relativ zum oberen Führungsbolzen 60 entlang der Achse A. Die relative Beweglichkeit der Aufnahmehülse ist erforderlich, um eine in die Aufnahmehülse 46 eingeleitet Schlagkraft auf das von der Aufnahme 46 aufgenommene, in Fig. 5 nicht dargestellte Implantat weiterzuleiten. Die Schlagkraft wird in die Aufnahme 46 mittels des koaxial zur Aufnahme 46 angeordneten, oberen Schlittens 58 eingeleitet.

Innerhalb des Gehäuses 50 ist etwas unterhalb des oberen Führungsbolzens 60 ein mittlerer Führungsbolzen 70 angeordnet. Der mittlere Führungsbolzen 70 ist ortsfest im Gehäuse 50 befestigt und erstreckt sich parallel zum oberen Führungsbolzen 60. Ein unterer Schlitten 72 ist mittels eines Kugellagers 74 entlang des mittleren Führungsbolzens 70, welcher den unteren Schlitten 72 durchgreift, geführt beweglich. Radial außen ist der mittlere Führungsbolzen 70 von einer Schraubenfeder 76 umgeben, welche sich einerseits am Gehäuse 50 und andererseits am unteren Schlitten 72 abstützt. Bei einer Verschiebung des unteren Schlittens 72 entlang des mittleren Führungsbolzens 70 in Fig. 5 nach links wird die Schraubenfeder 76 komprimiert. Gleichzeitig wird der untere Schlitten 72 in Fig. 5 nach rechts vorgespannt.

Der untere Schlitten 72 ist starr mit dem Pistolenabzug 54 gekoppelt. Bei einer Betätigung des Pistolenabzugs 54, d.h. bei einer Verschiebung des Pistolenabzugs 54 in Fig. 5 nach links, wird der untere Schlitten 72 daher ebenfalls nach links verschoben. In den Pistolenabzug 54 ist ein Kunststoffpfropfen 82 eingepreßt. Der Kunststoffpfropfen 82 gelangt bei eine Betätigung des Pistolenabzugs 54 in Anlage an den Pistolengriff 48 und begrenzt damit die Beweglichkeit des Pistolenabzugs 54.

Der Pistolenabzug 54 ist an seinem in Fig. 5 unteren Ende mit einem relativ zum Gehäuse 50 beweglichen, unteren Führungsbolzen 76 verbunden. Der untere Führungsbolzen 76 ist in einer im Pistolengriff 48 ausgebildeten Sacklochbohrung 78 entlang einer Achse B verschiebbar geführt. Zur Reduzierung des Reibungswiderstands ist in die Sacklochbohrung 78 eine Kunststoffhülse 80 eingepreßt, welche die Sacklochbohrung 78 radial innen auskleidet.

Innerhalb des Gehäuses 50 ist eine Koppeleinrichtung 84 vorgesehen. Die Koppeleinrichtung 84 gestattet es, den Betätigungsmechanismus, d.h. den Pistolenabzug 54, mit dem Schlagelement in Gestalt des oberen Schlittens 58 zu koppeln. Die Koppeleinrichtung 84 umfaßt einen in der Zeichenebene schwenkbaren Mitnehmer 86, eine Blattfeder 88 sowie ein Koppelelement 90.

Der Mitnehmer 86 ist schwenkbar am unteren Schlitten 72 befestigt und wird von der ebenfalls am unteren Schlitten 72 befestigten Blattfeder 88 in Richtung auf den oberen Schlitten 58 vorgespannt. Am oberen Schlitten 58 ist das mit dem Mitnehmer 86 zusammenwirkende Koppelelement 90 angeordnet.

Innerhalb des Gehäuses 50 ist außerdem eine Entkoppeleinrichtung 92 untergebracht, welche mit der Koppeleinrichtung 84 zusammenwirken und die Kopplung zwischen Pistolenabzug 54 und dem oberen Schlitten 58 aufheben kann. Die Entkoppeleinrichtung 92 umfaßt einen in das Gehäuse 50 ragenden Dorn 94. Um die Entkoppelschwelle verstellbar auszugestalten, ist der Dorn 94 entlang seiner Längsachse C beweglich. Zu diesem Zweck besitzt der Dorn 94 radial außen ein Außengewinde 96, welches mit einem komplementären Innengewinde 97 im Pistolenkörper 42 zusammenwirkt. Eine Kontermutter 98 gestattet die Arretierung des Dorns 94 in einer gewünschten, axialen Position. Der Dorn 94 ist an seinem der Dornspitze abgewandten Ende mit einem Innenmehrkant versehen, um zur Einstellung der Entkoppelschwelle den Angriff eines Werkzeugs am Dorn 94 zu ermöglichen.

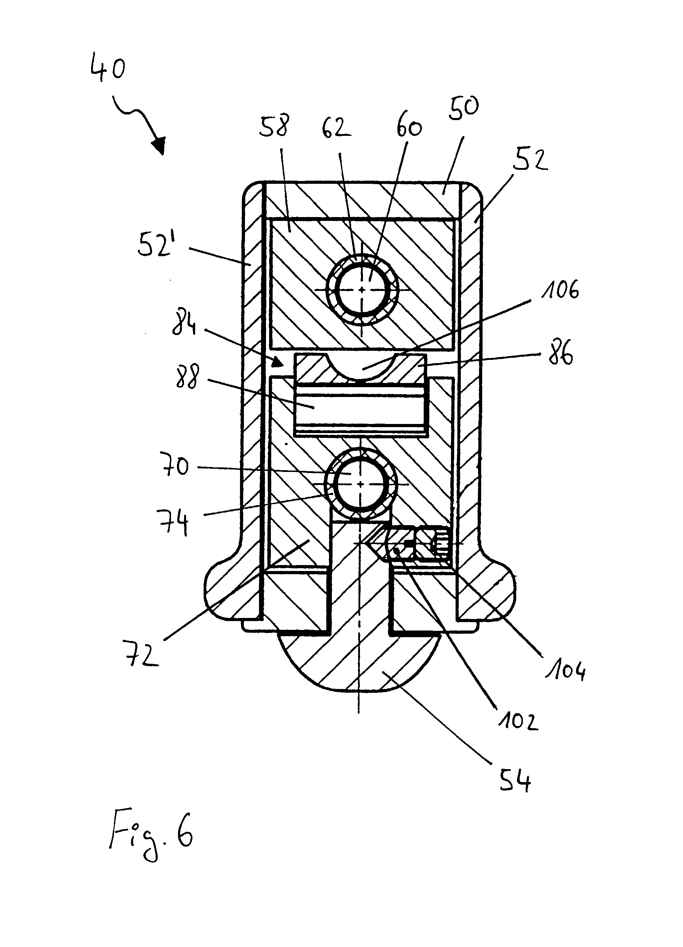

In Fig. 6 ist ein Querschnitt durch die in Fig. 4 abgebildete Eintreibvorrichtung 40 entlang der Schnittlinie IV-IV gemäß Fig. 5 dargestellt. Wie Fig. 6 entnommen werden kann, ist das Gehäuse 50 von zwei entfernbaren Gehäusedeckeln 52, 52' seitlich verschlossen. Innerhalb des Gehäuses 50 ist der Eintreibmechanismus mit dem entlang des oberen Führungsbolzens 60 geführten oberen Schlitten 58 und dem entlang des mittleren Führungsbolzens 70 geführten unteren Schlitten 72 angeordnet. Der untere Schlitten 72 ist mittels einer Schraube 102 mit dem Pistolenhandgriff 54 verbunden. Die Schraube 102 besitzt einen Innenmehrkant 104.

In Fig. 6 ist deutlich zu erkennen, daß der mit der Blattfeder 88 zusammenwirkende Mitnehmer 86 der Koppeleinrichtung 84 eine U-förmige Nut 106 besitzt. Mittels dieser Nut 106 wirkt die Koppeleinrichtung 84 mit dem Dorn 94 der Entkoppeleinrichtung zusammen. Die Nut 106 ist bezüglich der Zeichenebene derart angeordnet, daß der Mitnehmer 86 von der Spitze des Dorns 94 entgegen der Federkraft der Blattfeder 88 in Fig. 6 nach unten gedrückt werden kann.

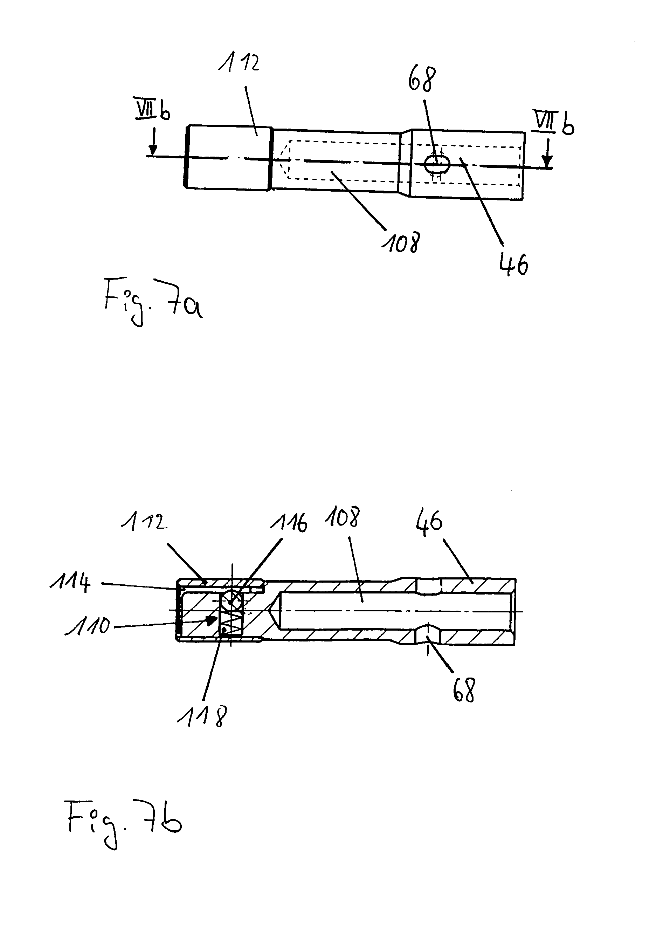

In den Fig. 7a und 7b ist die Aufnahme 46 der in Fig. 4 dargestellten Eintreibvorrichtung 40 abgebildet. Zu erkennen ist die ovale Öffnung 68, in welcher der in Fig. 5 dargestellte Querbolzen 66 beweglich geführt ist. Die Aufnahme 46 ist bereichsweise hülsenförmig ausgestaltet und besitzt eine Sacklochbohrung 108 zur Aufnahme eines Endbereichs des in Fig. 5 dargestellten oberen Führungsbolzens 60. An seinem der Sacklochbohrung 108 gegenüberliegenden Ende besitzt die Aufnahme 46 eine Hülse 112, welche den zylindrischen Grundkörper der Aufnahme 46 radial außen umgibt. Zwischen dem zylindrischen Grundkörper der Aufnahme 46 und der Hülse 112 ist ein ebener Schlitz 114 ausgebildet, welcher zu Aufnahme des Implantats dient. Der Schlitz 114 ist in der Schnittansicht gemäß Fig. 7b zu erkennen.

Im Bereich des Schlitzes 114 ist ein Selbsthaltemechanismus 110 angeordnet. Der Selbsthaltemechanismus 110 umfaßt eine Kugel 116 sowie eine die Kugel 116 in Richtung auf den Schlitz 114 vorspannende Feder 118. Die Kugel 116 wirkt mit dem in Fig. 2 dargestellten Schraubenloch 26 des Implantats 10 zusammen.

Nachfolgend wird die Funktionsweise der vorstehend unter Bezugnahme auf die Fig. 4 bis 7b beschriebenen Eintreibvorrichtung 40 näher erläutert.

In einem ersten Schritt wird das in den Fig. 1 bis 3 dargestellt Implantat 10 mit seinem das Schraubenloch 26 aufweisenden linken Auflagearm 22 in den Schlitz 114 der Aufnahme 46 eingeführt. Sobald das Schraubenloch 26 im Bereich der in Richtung auf den Schlitz 114 vorgespannten Kugel 116 liegt, greift die Kugel 116 in das Schraubenloch 26 ein und der Selbsthaltemechanismus 110 ist aktiviert. Da die Kugel 116 unter Vorspannung in das Schraubenloch des Implantats eingreift, kann das Implantat nicht mehr versehentlich aus dem Schlitz 114 gleiten.

Vorzugsweise wird das Implantat unmittelbar mittels der Eintreibvorrichtung 40 aus einem Implantatcontainer entnommen. Das Implantat muß folglich nicht unter Zuhilfenahme der Finger in den Schlitz 114 eingeführt werden. Die Handhabung des erfindungsgemäßen Implantatsystems aus Implantat und Eintreibvorrichtung 40 wird dadurch wesentlich verbessert.

Nachdem das Implantat in den Schlitz 114 eingeführt und dort mittels des Selbsthaltemechanismus 110 gegen ein Herausfallen gesichert ist, wird das gesicherte Implantat mittels der Eintreibvorrichtung 40 im Bereich des festzulegenden Knochendekkels oder Knochenfragments positioniert. Die Positionierung erfolgt derart, daß der in Fig. 1 dargestellte, rechte Auflagearm 24 des Implantats 10 auf der Oberfläche des Knochendeckels oder Knochenfragments aufliegt und die Spitze lateral den spongiösen oder kortikalen Bereich kontaktiert.

Im Anschluß daran wird der Pistolenabzug 54 mittels zweier Finger in Fig. 5 nach links bewegt. Von dieser Bewegung des Pistolenabzugs 54 wird auch der starr mit dem Pistolenabzug 54 gekoppelte untere Schlitten 52 erfaßt. Dieser bewegt sich folglich entgegen der Federkraft der Schraubenfeder 76 in Fig. 5 nach links. Aufgrund der Verschiebung des unteren Schlittens 72 in Fig. 5 nach links gelangt der Mitnehmer 86 in Kontakt mit dem Koppelelement 90 des oberen Schlittens 58. Sobald der Mitnehmer 84 am Koppelelement 90 anliegt, wird der obere Schlitten 58 vom Mitnehmer 86 mitgenommen und entgegen der Federkraft der Schraubenfeder 64 ebenfalls in Fig. 5 nach links verschoben.

Die Kopplung des unteren Schlittens 72 mit dem oberen Schlitten 58 wird vom Benutzer der Eintreibvorrichtung 40 dadurch zur Kenntnis genommen, daß die für die Betätigung des Pistolenabzugs 54 erforderliche Kraft ansteigt. Dieser Kraftanstieg ist darauf zurückzuführen, daß infolge der Kopplung von unterem Schlitten 72 und oberem Schlitten 58 nicht mehr nur die untere Schraubenfeder 76, sondern nunmehr zusätzlich die obere Schraubenfeder 84 komprimiert werden muß.

Nach einer gewissen Verschiebung der beiden Schlitten 72, 58 gelangt der Dorn 94 in Kontakt mit der in Fig. 6 dargestellten Nut 108 des Mitnehmers 86. Bei einer weiteren Verschiebung der beiden Schlitten 58, 72 in Fig. 5 nach links wird der Mitnehmer 86 nun von der Schrägfläche der Spitze des Dorns 94 um dessen in Fig. 5 dargestellte Schwenkachse 120 gegen die Federkraft der Blattfeder 88 nach unten geschwenkt. Bei einem Grenzschwenkwinkel wird schließlich die Kopplung zwischen dem Mitnehmer 86 und dem Koppelelement 90 des oberen Schlittens 58 abrupt aufgehoben. Infolge der Entkopplung von unterem Schlitten 72 und oberem Schlitten 58 wird der von der Schraubenfeder 64 in Fig. 5 nach rechts vorgespannte obere Schlitten 58 in Fig. 5 entlang des oberen Führungskolbens 60 nach rechts beschleunigt. Der beschleunigte obere Schlitten 58 gelangt schlagartig in Anlage mit der dem oberen Schlitten 58 zugewandten Stirnseite des entlang der Achse A geringfügig beweglichen Aufnehmers 46 und übt einen Schlag auf diesen aus. Dieser Schlag überträgt sich auf das im Schlitz 114 angeordnete Implantat 10, dessen Spitze 16 daraufhin lateral in den Knochendeckel oder das Knochenfragment eingetrieben wird. Während des Eintreibens wirkt der auf dem Knochendeckel oder Knochenfragment aufliegende Auflagearm 24 des Implantats 10 als Führung.

Sobald der obere Schlitten 58 vom unteren Schlitten 72 entkoppelt ist, kann der Pistolenabzug 54 wieder frei gegeben werden. Nach Freigabe des Pistolenabzugs 54 dehnt sich die komprimierte Schraubenfeder 76 wieder aus und der Pistolenabzug 54 wird von der Schraubenfeder 76 in die in Fig. 5 dargestellte Ausgangsposition zurückbewegt. Im Anschluß daran kann der vorstehend beschrieben Eintreibvorgang wiederholt werden, um erforderlichenfalls die Spitze 16 des Implantats 10 noch tiefer einzutreiben. Sobald der Fortsatz 14 lateral am Knochendeckel oder Knochenfragment anliegt, ist der Eintreibvorgang beendet. Der Fortsatz 14 fungiert dann als mechanischer Anschlag, der verhindert, daß die Spitze 16 zu tief in den Knochendeckel eindringt.

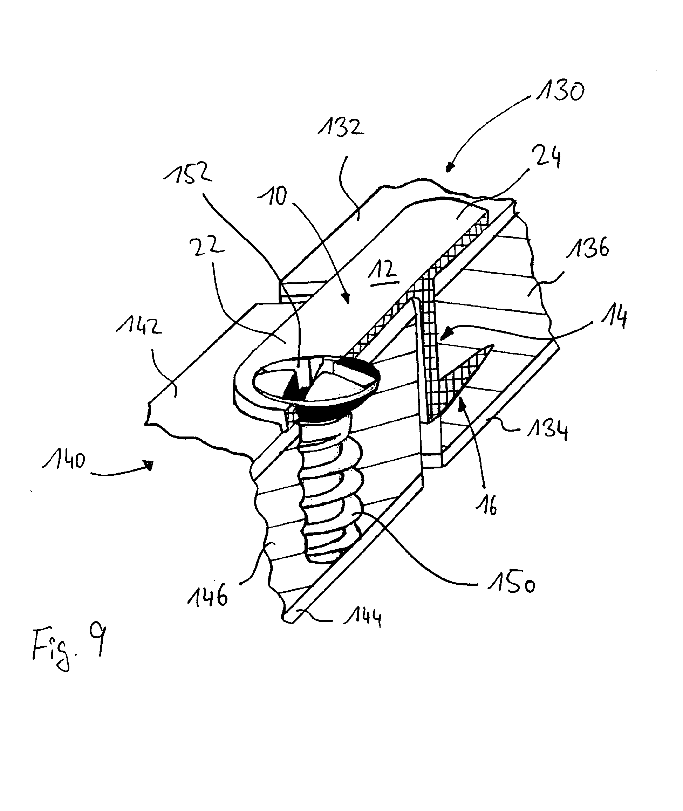

Üblicherweise müssen drei bis vier Implantate 10 gemäß den Fig. 1 bis 3 in einem Knochendeckel positioniert werden, um diesen zuverlässig im Schädelknochen festlegen zu können. Nachdem die erforderliche Anzahl von Implantaten in den Knochendeckel eingetrieben wurde, wird der Knochendeckel in der korrespondierenden Öffnung des Schädelknochens positioniert und mit je einer Knochenschraube pro Implantat am Schädelknochen befestigt. Dies ist in Fig. 9, welche weiter unten ausführlicher beschrieben wird, näher dargestellt.

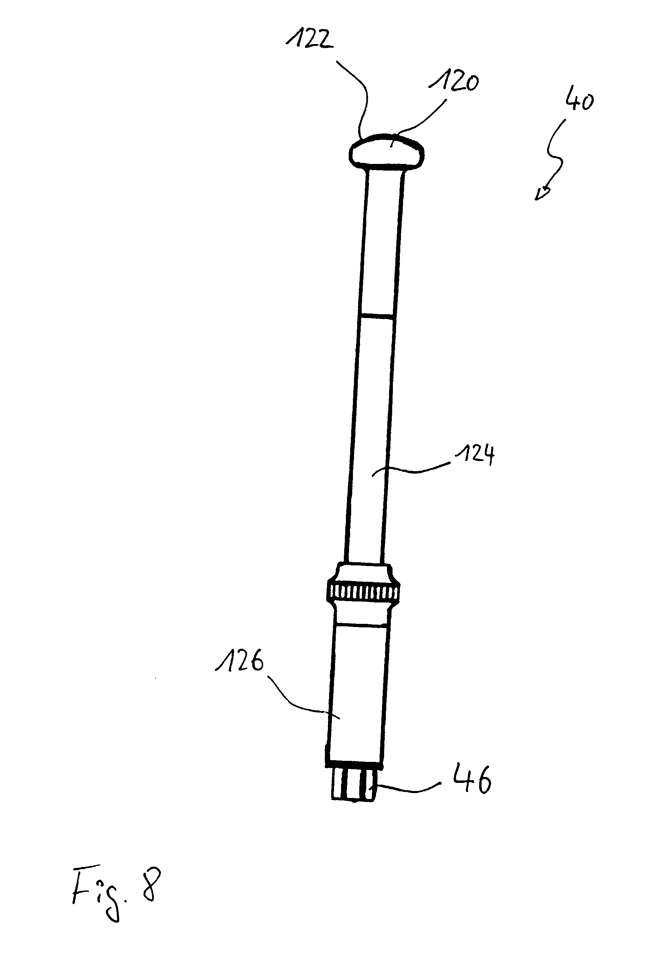

In Fig. 8 ist ein zweites Ausführungsbeispiel einer Eintreibvorrichtung 40 zum Eintreiben eines Implantats lateral in einen Knochendeckel oder ein Knochenfragment dargestellt. Die Eintreibvorrichtung 40 weist eine längliche Bauform auf und besitzt eine Aufnahme 46 für das Implantat sowie eine knaufartige Krafteinleitungsstruktur 120 mit einer sphärisch gekrümmten Krafteinleitungsfläche 122. Die Eintreibvorrichtung 40 umfaßt weiterhin einen im wesentlichen zylindrischen Körper 124, welcher kraftübertragend zwischen der Krafteinleitungsstruktur 120 und der Aufnahme 46 angeordnet ist. An seinem der Aufnahme 46 zugewandten Ende trägt der zylindrische Körper 124 ein Handstück 126, welches den zylindrischen Körper 124 radial außen umgibt. Das Handstück 126 ist in axialer Richtung unbeweglich mit dem Körper 124 gekoppelt. Die Aufnahme 46 besitzt den in den Fig. 7a und 7b dargestellten Selbsthaltemechanismus für das Implantat.

Zur Befestigung des Implantats an z.B. einer Knochenplatte wird dieses zunächst mittels des Selbsthaltemechanismus im Bereich der Aufnahme 46 fixiert. Anschließend wird das Implantat wie vorstehend beschrieben positioniert und eine Schlagkraft beispielsweise mittels eines Hammers in die Krafteinleitungsfläche 122 der Krafteinleitungsstruktur 120 eingeleitet. Die Schlagkraft überträgt sich von der Krafteinleitungsstruktur über den Körper 124 und die Aufnahme 46 auf das Implantat 10, welches daraufhin lateral in den Knochendeckel eingetrieben wird.

Fig. 9 zeigt einen Knochendeckel 130, welcher eine obere Knochentafel 132 (erste kortikale Schicht), eine untere Knochentafel 134 (zweite kortikale Schicht) sowie einen zwischen der oberen Knochentafel 132 und der unteren Knochentafel 134 angeordneten, spongiösen Bereich 136 besitzt. Das Implantat 10 wurde mittels der unter Bezugnahme auf die Fig. 4 bis 7b beschriebenen Eintreibvorrichtung 40 am Knochendeckel 130 befestigt. Die Spitze 16 des Implantats 10 wurde so tief in den spongiösen Bereich 136 des Knochendeckels 130 eingetrieben, daß der Fortsatz 14 lateral am Knochendeckel 130 anliegt.

Nach dem Befestigen einer Mehrzahl von Implantaten 10 am Knochendeckel 130 wurde der Knochendeckel in der entsprechenden Öffnung des Schädelknochens 140 positioniert. Dabei gelangt die Unterseite des Auflagearms 22 des Implantats 10 in Anlage mit der oberen Knochentafel 142 des Schädelknochens 140. Der Schädelknochen 140 besitzt ebenfalls eine spongiösen Bereich 146, welcher zwischen der oberen Knochentafel 142 und einer unteren Knochentafel 144 angeordnet ist.

Nach dem Positionieren des mit dem Implantat 10 versehenen Knochendeckels 130 in der Öffnung des Schädelknochens 140 muß der Knochendeckel 130 innerhalb der Öffnung des Schädelknochens 140 festgelegt werden. Zu diesem Zweck werden die Implantate 10 mittels Knochenschrauben 150 am Schädelknochen 140 befestigt. Dabei wird der sphärische Kopf 152 jeder Knochenschraube 150 vollständig im Schraubenloch des Implantats 10 versenkt.

Bei bikortikalen Knochen wie dem in Fig. 9 dargestellten Knochendeckel 130 ist es zweckmäßig, die Spitze 16 des Implantats 10 in den zwischen den beiden kortikalen Schichten angeordneten spongiösen Bereich einzutreiben. Das Eintreiben der Spitze 16 in eine kortikale Schicht ist jedoch ebenfalls möglich und bei monokortikalen Knochen sogar unabdingbar.

## Patentansprüche

1. Selbsthaltendes Implantat (10) zum Festlegen eines Knochendeckels (130) oder eines Knochenfragments, mit:
- einem Auflageelement (12) mit einer Oberseite (18) und einer dem Knochendeckel (130) oder dem Knochenfragment zugewandten Unterseite (20) und
- einem an der Unterseite (20) des Auflageelements (12) angeordneten Fortsatz (14), der mindestens eine, sich in Richtung auf den Knochendeckel (130) oder das Knochenfragment erstreckende Spitze (16) trägt, die zur selbsthaltenden Verankerung des Implantats lateral in den Knochendeckel (130) oder das Knochenfragment eintreibbar ist,
**dadurch gekennzeichnet, daß** das Auflageelement (12) zwei sich in entgegengesetzte Richtungen erstreckende Auflagearme (22, 24) umfaßt, wobei ein erster der beiden Auflagearme (22) mit einem Schädelknochen (140) und ein zweiter der beiden Auflagearme (24) mit dem Knochendeckel (130) oder dem Knochenfragment zusammenwirken kann.

2. Implantat nach Anspruch 1,
**dadurch gekennzeichnet, daß** der Fortsatz (14) sich im wesentlichen rechtwinkelig zum Auflageelement (12) erstreckt.

3. Implantat nach Anspruch 1 oder 2,
**dadurch gekennzeichnet, daß** das Auflageelement (12) eine streifenförmige Gestalt aufweist.

4. Implantat nach einem der Ansprüche 1 bis 3,
**dadurch gekennzeichnet, daß** die Unterseite (20) des Auflageelements (12) zumindest bereichsweise konkav oder sphärisch gekrümmt ist.

5. Implantat nach einem der Ansprüche 1 bis 4,
**dadurch gekennzeichnet, daß** die Spitze (16) an einem dem Auflagebereich (12) abgewandten Ende des Fortsatzes (14) angeordnet ist.

6. Implantat nach einem der Ansprüche 1 bis 5,
**dadurch gekennzeichnet, daß** die Spitze (16) eine dreieckige Gestalt besitzt.

7. Implantat nach einem der Ansprüche 1 bis 6,
**dadurch gekennzeichnet, daß** die Spitze (16) geschärfte Kanten besitzt.

8. Implantat nach einem der Ansprüche 1 bis 7,
**dadurch gekennzeichnet, daß** das Auflageelement (12) an seinem mit dem Schädelknochen zusammenwirkenden Ende ein Schraubenloch (26) aufweist.

9. Implantat nach Anspruch 8,
**dadurch gekennzeichnet, daß** das Auflageelement (12) eine in Richtung auf das Schraubenloch (26) zunehmende Stärke (d) besitzt.

10. Implantat nach Anspruch 8 oder 9,
**dadurch gekennzeichnet, daß** das Schraubenloch (26) innen sphärisch gekrümmt ist.

## Claims

1. A self-retaining implant (10) for attaching a bone cover (130) or a bone fragment, comprising:
- a support element (12) with an upper side (18) and a lower side (20) which faces the bone cover (130) or the bone fragment, and
- an extension (14), which is arranged on the lower side (20) of the support element (12) and supports at least one spike (16) that extends towards the bone cover (130) or bone fragment and can be driven laterally into the bone cover (130) or bone fragment for anchoring the implant in self-retaining fashion,
**characterized in that** the support element (12) comprises two support arms (22, 24) extending in opposite directions, a first of the two support arms (22) being adapted to cooperate with a skull bone (140) and a second of the two support arms (24) being adapted to cooperate with the bone cover (130) or the bone fragment.

2. The implant according to claim 1, **characterized in that** the extension (14) extends substantially at right angles to the support element (12).

3. The implant according to claim 1 or 2, **characterized in that** the support element (12) has a strip-like shape.

4. The implant according to one of claims 1 to 3, **characterized in that** the lower side (20) of the support element (12) is concave or spherically curved at least in sections.

5. The implant according to one of claims 1 to 4, **characterized in that** the spike (16) is arranged at an end of the extension (14) facing away from the support element (12).

6. The implant according to one of claims 1 to 5, **characterized in that** the spike (16) has a triangular shape.

7. The implant according to one of claims 1 to 6, **characterized in that** the spike (16) has sharpened edges.

8. The implant according to one of claims 1 to 7, **characterized in that**, at its end cooperating with the skull bone, the support element (12) comprises a screw hole.

9. The implant according to claim 8, **characterized in that** the support element (12) has a thickness (d) increasing in the direction of the screw hole (26).

10. The implant according to claim 8 or 9, **characterized in that** the screw hole (26) is spherically curved on the inside.

## Revendications

1. Implant auto-rétentif (10) pour la fixation d'un volet osseux (130) ou d'un fragment osseux, avec :
- un élément d'appui (12) avec une face supérieure (18) et un face inférieure (20) dirigée vers le volet osseux (130) ou le fragment osseux et
- un prolongement (14) disposé sur la face inférieure (20) de l'élément d'appui (12) qui porte au moins une pointe (16) s'étendant en direction du volet osseux (130) ou du fragment osseux qui peut être enfoncée latéralement dans le volet osseux (130) ou le fragment osseux pour l'ancrage auto-rétentif de l'implant,
**caractérisé en ce que** l'élément d'appui (12) comprend deux bras d'appui (22, 24) s'étendant dans des directions opposées, un premier des deux bras d'appui (22) pouvant coopérer avec un os crânien (140) et un second des deux bras d'appui (24) pouvant coopérer avec le volet osseux (130) ou le fragment osseux.

2. Implant selon la revendication 1,
**caractérisé en ce que** le prolongement (14) s'étend sensiblement à angles droits par rapport à l'élément d'appui (12).

3. Implant selon la revendication 1 ou 2,
**caractérisé en ce que** l'élément d'appui (12) présente la forme d'une bande.

4. Implant selon l'une quelconque des revendications 1 à 3,
**caractérisé en ce que** la face inférieure (20) de l'élément d'appui (12) présente une courbure concave ou sphérique au moins dans certaines zones.

5. Implant selon l'une quelconque des revendications 1 à 4,
**caractérisé en ce que** la pointe (16) est disposée à une extrémité du prolongement (14) opposée à la zone d'appui (12).

6. Implant selon l'une quelconque des revendications 1 à 5,
**caractérisé en ce que** la pointe (16) a une forme triangulaire.

7. Implant selon l'une quelconque des revendications 1 à 6,
**caractérisé en ce que** la pointe (16) présente des arêtes aiguisées.

8. Implant selon l'une quelconque des revendications 1 à 7,
**caractérisé en ce que** l'élément d'appui (12) comporte un trou de vis (26) à son extrémité coopérant avec l'os crânien.

9. Implant selon la revendication 8,
**caractérisé en ce que** l'élément d'appui (12) a une épaisseur (d) croissante dans la direction du trou de vis (26).

10. Implant selon la revendication 8 ou 9,
**caractérisé en ce que** le trou de vis (26) présente une courbure intérieure sphérique.
